# EUROPÄISCHE PATENTSCHRIFT

(11) **EP 0 924 195 B1**
(45) Veröffentlichungstag und Bekanntmachung des Hinweises auf die Patenterteilung: **03.09.2003**
(21) Anmeldenummer: 98123267.1
(22) Anmeldetag: 07.12.1998
(51) Int. Cl.: C07C 253/30, C07C 255/28

(54) **Verfahren zur Herstellung von Aminocyanacetamid**
Process for the preparation of aminocyanacetamide
Procédé de préparation d'aminocyanacétamide

(30) Priorität: 18.12.1997 CH 290797
(43) Veröffentlichungstag der Anmeldung: 23.06.1999
(73) Patentinhaber: EPROVA Aktiengesellschaft, CH-8200 Schaffhausen (CH)
(72) Erfinder: Müller, Hans Rudolf, 8207 Schaffhausen (CH); Bollinger, Heinrich, 8222 Beringen (CH); Schwaninger, Peter, 8203 Schaffhausen (CH); Kurz, Martin, 8200 Schaffhausen (CH)

(56) Entgegenhaltungen:
- EP-A- 0 342 616
- MILLER ET AL.: "Substituted Imidazoles of the Purines" JOURNAL OF AMERICAN CHEMISTRY SOCIETY, Bd. 74, 1952, Seiten 2892-2894, XP002100245
- J. MARCH: "Advanced Organic Chemistry" , JOHN WILEY & SONS XP002100246 * Seite 592 - 593, siehe 2-8 * * Seite 1218 - 1219, siehe 9-51 *

## Beschreibung

Die Erfindung betrifft ein neues, technisch einsetzbares Verfahren zur Herstellung von Aminocyanacetamid. Aminocyanacetamid ist ein interessantes Zwischenprodukt zur Herstellung von z. B. Imidazolen, Pyrazinen, Purinen oder Pteridinen.

Zur Herstellung von Aminocyanacetamid sind verschiedene Verfahren bekannt. Speziell zu erwähnen sind dabei Taylor et al., die in *J. Am. Chem. Soc*., 76, 6080 (1954) ein Verfahren zur Herstellung von Aminocyanacetamid, ausgehend von Hydroxyiminocyanacetamid, durch Hydrierung mit Aluminiumamalgam beschreiben und verschiedene Verfahren zur Herstellung von Aminocyanacetamid, ausgehend von Cyanessigsäureestern über Aminocyanessigester wie EP 0 342 616, aus der die Herstellung von Aminocyanacetamid durch Nitrosierung eines Cyanessigsäureesters mittels eines Alkalinitrits, Hydrierung des entstandenen Hydroxyiminocyanessigsäureesters mit Platin/Wasserstoff und anschliessender Freisetzung von Aminocyanacetamid mit wässrigem Ammoniak hervorgeht. Von der Problematik der Verwendung eines Quecksilber-Katalysators her ist das Verfahren von Taylor et al. technisch nicht zu verwenden. Verfahren zur Herstellung von Aminocyanacetamid, ausgehend von Cyanessigsäureestern über Aminocyanessigester wie EP 0 342 616, beschreiben technisch aufwendige Verfahren, die auf der umständlichen Umsetzung einer über eine Ester-Funktion geschützten Cyanessigsäure zu einem Aminocyanessigester und anschliessender Freisetzung von Aminocyanacetamid basieren. Miller et al., beschreibt in *J. Am. Chem. Soc*., 74, 2892-2894 (1952) ein Verfahren zur Herstellung von Aminocyanacetamid durch Umsetzung von Cyanacetamid in wässriger Essigsäure und anschliessender Reduktion des Nitrosocyanacetamids mit Raney-Nickel zu Aminocyanacetamid. Bei der Nitrosierung in wässriger Essigsäure werden grössere Mengen Nebenprodukte gebildet und mit Natriumacetat entsteht des unerwünschte, weil mit nicht kontrollierbarer Exothermie kristallisierende, sehr feine Halb-Natrium-Salz. Die nachfolgende Reduktion mit Raney-Nickel zeigt starke Tendenzen zur Überhydrierung unter Bildung einer ganzen Palette von Nebenprodukten.

Mit dem vorliegenden Verfahren kann nun erstmals ein technisch einsetzbarer Prozess zur direkten Herstellung von Aminocyanacetamid verwendet werden. Das Verfahren basiert auf der Umsetzung von Cyanacetamid mit Nitriten bei einem pH-Wert um 2 zu Nitrosocyanacetamid und der darauf folgenden katalytischen Hydrierung von Nitrosocyanacetamid zu Aminocyanacetamid.

Cyanacetamid ist ein grosstechnisch verfügbares Ausgangsmaterial. Cyanacetamid wird mit Nitriten bei einem pH-Wert um 2 zu Nitrosocyanacetamid nitrosiert. Für die Nitrosierung wird vorzugsweise ein Alkalinitrit verwendet, das vorzugsweise zu einer sauren Lösung oder Suspension von Cyanacetamid gegeben wird. Dabei wird der pH-Wert während der Nitrosierung vorzugsweise um 2 gehalten. Dieses Vorgehen verhindert die Bildung grösserer Mengen Nebenprodukte, reduziert die Menge entstehender nitroser Gase und ergibt ein gut zentrifugierbares Produkt. Übliche Nitrite für die Nitrosierung sind Alkalinitrite vorzugsweise Natriumnitrit. Das Nitrit wird in einer Menge von 1-5 Aequivalenten bezogen auf Cyanacetamid, vorzugsweise 1-2 Aequivalenten im Bezug zum Cyanacetamid eingesetzt. Die Nitrosierung erfolgt in sauren Medien, vorzugsweise in konzentrierten, starken Mineralsäuren, im speziellen in Salzsäure bei einem pH-Wert um 2. Die Reaktion findet in einer konzentrierten Lösung oder vorzugsweise in einer Suspension, bei Temperaturen zwischen 0°C und 50°C, vorzugsweise zwischen 0°C und 5°C statt.

Das so erhaltene Nitrosocyanacetamid wird mit einer katalytischen Hydrierung zu Aminocyanacetamid umgesetzt. Die Hydrierung erfolgt mit Wasserstoff in Gegenwart eines Katalysators. Geeignete Katalysatoren sind vorzugsweise Edelmetallkatalysatoren wie Platin, das in Mengen von 1% bis 20% auf den üblichen Trägermaterialien wie Kohle, Aluminiumoxid, Siliciumdioxid, Bariumsulfat oder Calciumcarbonat fein verteilt ist, oder Platinoxid. Bevorzugt als Katalysator wird Platin in einer Menge von 2% bis 10% auf Kohle eingesetzt.
Zweckmässig wird der Katalysator in Mengen von 1% bis 30% bezogen auf das Nitrosocyanacetamid, vorzugsweise 2% bis 10% bezogen auf das Nitrosocyanacetamid verwendet.

Die Hydrierung erfolgt entweder in wässrigen Medien, wie in Wasser, oder vorzugsweise in vorwiegend nichtwässrigen Medien, wie in niedrigen, mit Wasser mischbaren Alkoholen wie Methanol oder Ethanol, in niedrigen, mit Wasser mischbaren Carbonsäuren wie Ameisensäure oder Essigsäure, oder in Tetrahydrofuran. Zur Abpufferung von wässrigen Hydrierlösungen kann wenig Natriumhydrogenphthalat zugefügt werden. Weitere gebräuchliche Zusatzstoffe wie Eisensulfat zur Verhinderung entstehender Blausäuregase, Raney-Nickel zur Unterdrückung von Katalysatorvergiftungen, usw. können der Hydrierlösung beigefügt werden.

Die Verwendung von wasserhaltigen Katalysatoren ergibt beim Arbeiten auch in nichtwässrigen Medien keine Probleme. Die eingesetzten Katalysatoren können nach erfolgter Reaktion einfach wiederaufgearbeitet und für die nächsten Reaktionen eingesetzt werden.

Die Hydrierung erfolgt bei einem Druck von1 bar bis 100 bar, vorzugsweise 1 bar bis 10 bar und bei Temperaturen zwischen 0°C und 80°C, vorzugsweise zwischen 20°C und 50°C. Abhängig von Druck, Temperatur, Medium und Katalysatormenge kann die Hydrierzeit zwischen 30 Minuten und 20 Stunden variieren.

Nach erfolgter Hydrierung in wässrigen Medien kann der pH-Wert der Reaktionslösung durch die Zugabe einer Säure wie Essigsäure abgesenkt werden. Dieses Vorgehen verhindert die Bildung grösserer Mengen von Nebenprodukten.

### Beispiele zur Illustrierung der Erfindung

### Beispiel 1

### Herstellung von Nitrosocyanacetamid

In einem Reaktionsgefäss werden 33.5 kg Cyanacetamid in 78 l Wasser vorgelegt und mit 0.1 kg Salzsäure 37% auf pH 2.3 gestellt. Dann wird über 7 Stunden eine Lösung von 28.7 kg Natriumnitrit in 45 l Wasser zudosiert. Dabei wird der pH-Wert der Reaktionslösung durch Zudosieren von insgesamt 36 kg Salzsäure 37% zwischen 1.5 und 2.5 gehalten. Die Temperatur der Reaktionslösung wird auf unter 40°C gehalten. Aus der während der Zugabe der Natriumnitrit-Lösung entstandenen klaren gelbroten Lösung kristallisiert bereits vor Beendigung der Zugabe Nitrosocyanacetamid aus. Über Nacht wird die gelbe Suspension bei Raumtemperatur gerührt und anschliessend auf 5°C abgekühlt.
Der so erhaltene weisse, dicke Kristallisationsbrei wird bei 5°C abzentrifugiert mit Wasser nachgewaschen und bei 50°C am Vakuum getrocknet. Man erhält 33.9 kg Nitrosocyanacetamid mit einem Gehalt von 99.1.%, bestimmt mittels Titration. Die Indentifikation der Substanz erfolgte mittels IR- und NMR-Messungen. Ein Vergleich dieser Spektren mit einer Referenzsubstanz zeigte identische Werte.

### Beispiel 2

### Herstellung von Nitrosocyanacetamid

In einem Reaktionsgefäss werden bei 0°C 250 ml 37% Salzsäure (3 Mol) vorgelegt und mit 171.6 g Cyanacetamid (2 Mol) versetzt. Unter Rühren wird darauf innert 5-7 Stunden eine Lösung von 156.0 Natriumnitrit (2.26 Mol) in 600 ml Wasser zugetropft. Die Temperatur wird dabei bei 0°C bis 3°C gehalten.
Das entstandene weisse Kristallisat wird abgenutscht, mit Wasser nachgewaschen und bei 40°C am Vakuum getrocknet. Man erhält 142.6 g Nitrosocyanacetamid mit einem Gehalt von 97.4%, bestimmt mittels HPLC. Der Anteil an Cyanacetamid im so erhaltenen Produkt beträgt 0.3%, bestimmt mittels HPLC.

### Beispiel 3

### Herstellung von Nitrosocyanacetamid

2.0 kg Cyanacetamid und 1.8 kg Natriumnitrit werden in 4.3l Wasser vorgelegt. Nach dem Erwärmen auf 20°C werden innert 6 Stunden 2.4 l 37% Salzsäure zugetropft. Die Temperatur wird dabei bei 40°C bis 50°C gehalten. Nach beendeter Zugabe wird die entstandene Suspension bei 35°C bis 40°C noch 40-50 Minuten nachgerührt und danach auf 10°C gekühlt.
Das entstandene weisse Kristallisat wird abgenutscht, mit Wasser nachgewaschen und bei 40°C am Vakuum getrocknet. Man erhält 2.5 kg Nitrosocyanacetamid mit einem Gehalt von 90.7%.

Die so erhaltenen 2.5 kg Nitrosocyanacetamid werden in 7.1l Isopropanol zum Sieden erwärmt und mit 70 ml Wasser versetzt. Nach 2 Stunden wird die Suspension klarfiltriert. Der erhaltene Rückstand wird mit 800 ml heissem Isopropanol überwaschen. Nach dem Einengen des Filtrates auf ca. 4l wird die gelbliche Suspension über Nacht bei -20°C gehalten.
Das entstandene weisse Kristallisat wird abgenutscht und bei 40°C am Vakuum getrocknet. Man erhält 2.0 kg Nitrosocyanacetamid mit einem Gehalt von 100.0% und einem Zersetzungspunkt von ca. 170°C. Der Anteil an NaCl im so erhaltenen Produkt beträgt weniger als 0.5%.

### Beispiel 4

### Herstellung von Nitrosocyanacetamid

7.5 l 37% Salzsäure werden vorgelegt, mit 5.15 kg Cyanacetamid versetzt. Unter Rühren wird darauf innert 3 Stunden eine Lösung von 4.68 kg Natriumnitrit in 1.8 l Wasser zugetropft. Die Temperatur wird dabei auf 5°C gehalten.
Das entstandene weisse Kristallisat wird abgenutscht, mit Wasser nachgewaschen und bei 40°C am Vakuum getrocknet. Man erhält 5.47 kg Nitrosocyanacetamid mit einem Gehalt von 98.7%, bestimmt mittels HPLC. Der Chloridgehalt im so erhaltenen Produkt beträgt 0.4%.

### Beispiel 5

### Herstellung von Aminocyanacetamid

31 g Nitrosocyanacetamid (0.27 Mol) werden in einem Stahlautoklav in 700 ml Methanol vorgelegt und mit 20 g Platinkatalysator 5% auf Kohle mit 50% Wasser versetzt. Nach dem Spülen mit Stickstoff und Wasserstoff wird bei Raumtemperatur und 2 bar Wasserstoffdruck während 14 Stunden hydriert
Nach dem Begasen wird der Autoklaveninhalt abgenutscht und das erhaltene hellgelbe Filtrat am Vakuum bis zur Kristallisation eingeengt. Das Kristallisat wird in 250 ml Isopropanol aufgeschlämmt, abgenutscht und bei 40°C am Vakuum getrocknet. Man erhält 23 g leicht hellbeiges Aminocyanacetamid mit einem Gehalt von 84.1%, bestimmt mittels HPLC und einem Zersetzungspunkt von ca. 120°C.

### Beispiel 6

### Herstellung von Aminocyanacetamid

Analog zu Beispiel 5 wurden Hydrierungen in den folgenden Medien und mit den aufgeführten Katalysatoren durchgeführt.

| **Lösungsmittel** | **Katalysatormenge** | **Pt-Anteil** | **Temperatur** | **Hydrierdauer** | **Ausbeute** | **Gehalt** |
|---|---|---|---|---|---|---|
| a Tetrahydrofuran | 5 g | 5.0% | RT | 27 Std. | 52.7% | 88.0% |
| b Ethanol | 5 g | 5.0% | RT | 50 Std. | 79.2% | 79.1% |
| c Methanol | 20 g | 2.5% | RT | 17 Std. | 87.1% | 80.8% |
| d Essigsäure 40% | 7.75 g | 5.0% | 25°C | 10 Std. | 66.2% | 94.2% |
| e Essigsäure 40% | 7.75 g | 5.0% | 24°C | 8 Std. | 69.4% | 97.5% |
| f Essigsäure 40% | 7.75 g | 5.0% | 25°C | 7 Std. | 72.6% | 99.2% |
| g Wasser | 7.75 g | 5.0% | 24°C | 6 Std. | 66.5% | 89.6% |
| h Essigsäure 0.25% | 7.75 g | 5.0% | 24°C | 5 Std. | 75.7% | 97.0% |
| i Wasser | 7.75 g | 5.0% | 24°C | 6 Std. | 68.1% | 98.9% |
| j Wasser | 7.75 g | 5.0% | 10°C | 9 Std. | 73.8% | 99.9% |
| k Wasser | 7.75 g | 5.0% | 12°C | 9 Std. | 69.0% | 99.9% |
| l Wasser | 7.75 g | 5.0% | 11°C | 8 Std. | 72.1% | 99.5% |
| m Wasser | 7.75 g | 5.0% | 12°C | 17 Std. | 81.4% | 96.0% |
| n Wasser | 7.75 g | 5.0% | 12°C | 20 Std. | 77.8% | 88.8% |
| o Wasser | 7.75 g¹⁾ | 5.0% | 23°C | 9 Std. | 74.4% | 85.6% |
| p Wasser | 7.75 g¹⁾ | 5.0% | 23°C | 7 Std. | 78.5% | 99.5% |
| q Wasser | 7.75 g¹⁾ | 5.0% | 23°C | 7 Std. | 80.1% | 99.9% |
| r Wasser | 7.75 g¹⁾ | 5.0% | 23°C | 7 Std. | 83.0% | 99.9% |
| s Wasser | 7.75 g¹⁾ | 5.0% | 23°C | 7 Std. | 81.9% | 99.9% |
| t Wasser | 7.75 g¹⁾ | 5.0% | 23°C | 7 Std. | 82.6% | 99.9% |

| | | | | | | |
|---|---|---|---|---|---|---|
| ¹⁾ In den Beispielen o bis t wurde jeweils der im vorangegangenen Ansatz verwendete Katalysator eingesetzt. Die Katalysatoraufarbeitung bestand jeweils im Waschen mit Wasser/verdünnter Salzsäure und anschliessendem Trocknen bei 130°C. | | | | | | |

### Beispiel 7

### Herstellung von Aminocyanacetamid

200 g Nitrosocyanacetamid (1.77 Mol) werden in einem Stahlautoklav in 2l Wasser vorgelegt und mit 49.45 g Platinkatalysator 5% auf Kohle versetzt. Nach dem Spülen mit Stickstoff und Wasserstoff wird bei 24°C und 2 bar Wasserstoffdruck während 8 Stunden hydriert.
Nach dem Begasen wird der Hydrierlösung 117 ml Essigsäure zugefügt. Die klarfiltrierte gelbliche Hydrierlösung wird am Vakuum bei 50°C bis zur Kristallisation eingeengt. Das Kristallisat wird in 450 ml Isopropanol versetzt und über Nacht bei 0°C gehalten.
Das entstandene Kristallisat wird abgenutscht, mit kaltem Isopropanol nachgewaschen und bei 40°C am Vakuum getrocknet. Man erhält 136.7 sehr schwach hellgelbliches Aminocyanacetamid mit einem Gehalt von 99.7%, bestimmt mittels Titration und einem Gehalt an Aminomalonsäurediamid von 0.26%, bestimmt mittels HPLC.
100 g so erhaltenes rohes Aminocyanacetamid wird in 240 ml Wasser von 85°C gelöst und rasch auf 0°C abgekühlt. Das so erhaltene Kristallisat wird abgenutscht, mit kaltem Isopropanol nachgewaschen und bei 40°C am Vakuum getrocknet. Man erhält 79.2 g farbloses Aminocyanacetamid mit einem Gehalt von 100.0%, bestimmt mittels HPLC.
Analog werden 20 g vom erhaltenen rohen Aminocyanacetamid in einem Gemisch von 200 ml Wasser und 600 ml Isopropanol bei 20°C gelöst und rasch auf 0°C abgekühlt. Das so erhaltene Kristallisat wird abgenutscht, mit kaltem Wasser-lsopropanol-Gemisch nachgewaschen und bei 40°C am Vakuum getrocknet. Man erhält 11.6 g farbloses Aminocyanacetamid mit einem Gehalt von 100.0%, bestimmt mittels HPLC.
So erhaltenes Produkt besitzt bei Raumtemperatur eine Löslichkeit in Methanol von 1.65 g/100 ml und in Ethanol von 0.85 g/100 ml.

### Beispiel 8

### Herstellung von Aminocyanacetamid

8.5 kg Platinkatalysator 5% auf Kohle (ca. 50% Wasser) werden in einem inertisierten Hydrierreaktor vorgelegt und mit 119 kg Methanol versetzt. Zu dieser Suspension werden 14.7 kg Nitrosocyanacetamid (130 Mol) zugefügt. Nach dem Spülen mit Stickstoff und Wasserstoff wird bei 40-45°C und 2 bar Wasserstoffdruck während 11 Stunden hydriert.
Nach dem Begasen wird die Hydrierlösung klarfiltriert und das Filtrat über Nacht auf -18°C gekühlt.
Das entstandene Kristallisat wird abzentrifugiert, mit kaltem Ethanol nachgewaschen und bei 40°C am Vakuum getrocknet. Man erhält 8.3 kg leicht gelbliches Aminocyanacetamid mit einem Gehalt von 99.2%, bestimmt mittels HPLC und einem Gehalt an Nitrosocyanacetamid von 3.5%, bestimmt mittels HPLC.
Durch Umkristallisation analog zu Beispiel 7 erhält man farbloses Aminocyanacetamid mit einem Gehalt von 100.0%, bestimmt mittels HPLC und einem Gehalt an Nitrosocyanacetamid von < 0.1%, bestimmt mittels HPLC.

## Patentansprüche

1. Verfahren zur Herstellung von Aminocyanacetamid, **dadurch gekennzeichnet, dass** Cyanacetamid mit Nitriten bei einem pH-Wert um 2 zu Nitrosocyanacetamid und dieses durch eine katalytischen Hydrierung zu Aminocyanacetamid umgesetzt wird.

2. Verfahren nach Anspruch 1, **dadurch gekennzeichnet, dass** zur Herstellung des Nitrosocyanacetamides Cyanacetamid in einem sauren Medium vorgelegt und das Nitrit dazugegeben wird.

3. Verfahren nach Anspruch 1 oder 2, **dadurch gekennzeichnet, dass** bei der Nitrosierung von Cyanacetamid zu Nitrosocyanacetamid der pH-Wert konstant gehalten wird.

4. Verfahren nach Anspruch 1, 2 oder 3, **dadurch gekennzeichnet, dass** als Nitrit ein Alkalinitrit, vorzugsweise Natriumnitrit eingesetzt wird.

5. Verfahren nach Anspruch 1, 2, 3 oder 4, **dadurch gekennzeichnet, dass** das Nitrit in einer Menge von 1-5 Aequivalenten bezogen auf Cyanacetamid, vorzugsweise 1-2 Aequivalenten bezogen auf Cyanacetamid eingesetzt wird.

6. Verfahren nach Anspruch 1, 2, 3, 4 oder 5, **dadurch gekennzeichnet, dass** Cyanacetamid in einer konzentrierten Lösung oder vorzugsweise in einer Suspension mit Nitriten zu Nitrosocyanacetamid umgesetzt wird.

7. Verfahren nach Anspruch 1, 2, 3, 4, 5 oder 6, **dadurch gekennzeichnet, dass** Cyanacetamid bei Temperaturen zwischen 0°C und 50°C, vorzugsweise zwischen 0°C und 5°C mit Nitriten zu Nitrosocyanacetamid umgesetzt wird.

8. Verfahren nach Anspruch 1, **dadurch gekennzeichnet, dass** Nitrosocyanacetamid durch eine katalytische Hydrierung in wässrigen Medien zu Aminocyanacetamid umgesetzt wird.

9. Verfahren nach Anspruch 1, **dadurch gekennzeichnet, dass** Nitrosocyanacetamid durch eine katalytische Hydrierung in nichtwässrigen Medien, vorzugsweise in niedrigen, mit Wasser mischbaren Alkoholen wie Methanol oder Ethanol, in niedrigen, mit Wasser mischbaren Carbonsäuren wie Ameisensäure oder Essigsäure, oder in Tetrahydrofuran zu Aminocyanacetamid umgesetzt wird.

10. Verfahren nach Anspruch 9, **dadurch gekennzeichnet, dass** Nitrosocyanacetamid durch eine katalytische Hydrierung in Methanol zu Aminocyanacetamid umgesetzt wird.

11. Verfahren nach Anspruch 1, 8, 9 oder 10, **dadurch gekennzeichnet, dass** als Katalysator für die katalytische Hydrierung von Nitrosocyanacetamid zu Aminocyanacetamid Edelmetallkatalysatoren wie Platin, das in Mengen von 1% bis 20% auf den üblichen Trägermaterialien wie Kohle, Aluminiumoxid, Siliciumdioxid, Bariumsulfat oder Calciumcarbonat fein verteilt ist, oder Platinoxid eingesetzt wird.

12. Verfahren nach Anspruch 1, 8, 9, 10 oder 11, **dadurch gekennzeichnet, dass** als Katalysator für die katalytische Hydrierung von Nitrosocyanacetamid zu Aminocyanacetamid Platin in einer Menge von 2% bis 10% auf Kohle eingesetzt wird.

13. Verfahren nach Anspruch 1, 8, 9, 10, 11 oder 12, **dadurch gekennzeichnet, dass** der Katalysator für die katalytische Hydrierung von Nitrosocyanacetamid zu Aminocyanacetamid in Mengen von 1% bis 30% bezogen auf das Nitrosocyanacetamid, vorzugsweise 2% bis 10% bezogen auf das Nitrosocyanacetamid eingesetzt wird.

14. Verfahren nach Anspruch 1, 8, 9, 10, 11, 12 oder 13, **dadurch gekennzeichnet, dass** Nitrosocyanacetamid durch eine katalytische Hydrierung mit Wasserstoff bei 1 bar bis 100 bar, vorzugsweise bei 1 bar bis 10 bar und bei Temperaturen zwischen 0°C und 80°C, vorzugsweise zwischen 20°C und 50°C zu Aminocyanacetamid umgesetzt wird.

## Claims

1. Method of producing aminocyanoacetamide, **characterized in that** it comprises reacting cyanoacetamide with nitrites, at a pH of about 2, to form nitrosocyanoacetamide and subjecting the latter to catalytic hydrogenation to form aminocyanoacetamide.

2. Method according to Claim 1, **characterized in that** the cyanoacetamide is placed in an acid medium and the nitrite is added thereto in order to form the nitrosocyanoacetamide.

3. Method according to Claim 1 or 2, **characterized in that** the pH is held constant during the nitrosylation of cyanoacetamide to form nitrosocyanoacetamide.

4. Method according to Claim 1, 2 or 3, **characterized in that** the nitrite is an alkali nitrite, preferably sodium nitrite.

5. Method according to Claim 1, 2, 3 or 4, **characterized in that** the nitrite is used in an amount of 1-5 equivalents with respect to the cyanoacetamide, preferably 1-2 equivalents with respect to the cyanoacetamide.

6. Method according to Claim 1, 2, 3, 4 or 5, **characterized in that** the cyanoacetamide is reacted with nitrites to form nitrosocyanoacetamide in a concentrated solution, or preferably in a suspension.

7. Method according to Claim 1, 2, 3, 4, 5 or 6, **characterized in that** cyanoacetamide is reacted with nitrites to form nitrosocyanoacetamide at temperatures between 0°C and 50°C, preferably between 0°C and 5°C.

8. Method according to Claim 1, **characterized in that** the nitrosocyanoacetamide is reacted to form aminocyanoacetamide by catalytic hydrogenation in aqueous media.

9. Method according to Claim 1, **characterized in that** the nitrosocyanoacetamide is reacted to form aminocyanoacetamide by catalytic hydrogenation in nonaqueous media, preferably in low-molecular-weight water-miscible alcohols such as methanol or ethanol, in low-molecular-weight water-miscible carboxylic acids such as formic acid or acetic acid, or in tetrahydrofuran.

10. Method according to Claim 9, **characterized in that** the nitrosocyanoacetamide is reacted to form aminocyanoacetamide by catalytic hydrogenation in methanol.

11. Method according to Claim 1, 8, 9 or 10, **characterized in that** the catalyst for hydrogenation of nitrosocyanoacetamide to form aminocyanoacetamide is platinum oxide or a finely divided noble metal catalyst such as platinum in amounts of 1% to 20% on a customary support selected from the group consisting of carbon, alumina, silica, barium sulphate and calcium carbonate.

12. Method according to Claim 1, 8, 9, 10 or 11, **characterized in that** the catalyst for hydrogenation of nitrosocyanoacetamide to form aminocyanoacetamide is platinum in an amount of 2% to 10% on carbon.

13. Method according to Claim 1, 8, 9, 10, 11 or 12, **characterized in that** in the catalytic hydrogenation of nitrosocyanoacetamide to form aminocyanoacetamide, the catalyst is used in amounts of 1% to 30% with respect to nitrosocyanoacetamide, preferably 2% to 10% with respect to nitrosocyanoacetamide.

14. Method according to Claim 1, 8, 9, 10, 11, 12 or 13, **characterized in that** nitrosocyanoacetamide is reacted to form aminocyanoacetamide by catalytic hydrogenation with hydrogen at 1 bar to 100 bar, preferably at 1 bar to 10 bar, and at temperatures between 0°C and 80°C, preferably between 20°C and 50°C.

## Revendications

1. Procédé de préparation d'aminocyanacétamide, **caractérisé en ce que** du cyanacétamide réagit avec un nitrite à un pH de 2 pour former du nitrosocyanacétamide et celui-ci est transformé par une réaction d'hydrogénation catalytique en aminocyanacétamide.

2. Procédé selon la revendication 1, **caractérisé en ce que** le cyanacétamide est placé dans un milieu acide et le nitrite est ajouté à celui-ci pour former le nitrosocyanacétamide.

3. Procédé selon la revendication 1 ou 2, **caractérisé en ce que** le pH est maintenu constant au cours de la nitrosation du cyanacétamide en nitrosocyanacétamide.

4. Procédé selon la revendication 1, 2 ou 3, **caractérisé en ce que** le nitrite utilisé est un nitrite de métal alcalin, de préférence un nitrite de sodium.

5. Procédé selon la revendication 1, 2, 3 ou 4, **caractérisé en ce que** la quantité de nitrite utilisé est de 1-5 équivalents par rapport au cyanacétamide, de préférence de 1-2 équivalents par rapport au cyanacétamide.

6. Procédé selon la revendication 1, 2, 3, 4 ou 5, **caractérisé en ce que** du cyanacétamide réagit, en solution concentrée ou de préférence en suspension, avec un nitrite pour former du nitrosocyanacétamide.

7. Procédé selon la revendication 1, 2, 3, 4, 5 ou 6, **caractérisé en ce que** du cyanacétamide réagit avec un nitrite pour former un nitrosocyanacétamide à une température comprise entre 0°C et 50°C, de préférence entre 0°C et 5°C.

8. Procédé selon la revendication 1, **caractérisé en ce que** du nitrosocyanacétamide est transformé en aminocyanacétamide par réaction d'hydrogénation catalytique en milieu aqueux.

9. Procédé selon la revendication 1, **caractérisé en ce que** du nitrosocyanacétamide est transformé en aminocyanacétamide par réaction d'hydrogénation catalytique en milieu non aqueux, de préférence dans un alcool inférieur miscible dans l'eau par exemple le méthanol ou l'éthanol, dans un acide carboxylique inférieur miscible dans l'eau par exemple l'acide formique ou l'acide acétique, dans un ester d'un acide carboxylique inférieur par exemple l'acétate d'éthyle, ou dans le tétrahydrofurane.

10. Procédé selon la revendication 9, **caractérisé en ce que** du nitrosocyanacétamide est transformé en aminocyanacétamide par une réaction d'hydrogénation catalytique dans du méthanol.

11. Procédé selon la revendication 1, 8, 9 ou 10, **caractérisé en ce que** le catalyseur utilisé pour la réaction d'hydrogénation catalytique du nitrosocyanacétamide en aminocyanacétamide est un oxyde de platine ou un catalyseur à base de métal précieux, par exemple le platine, finement divisé dans une quantité de 1% à 20% par rapport à un support choisi dans le groupe comprenant le carbone, l'oxyde d'aluminium, la silice, le sulfate de baryum ou le carbonate de calcium.

12. Procédé selon la revendication 1, 8, 9, 10 ou 11, **caractérisé en ce que** le catalyseur utilisé pour la réaction d'hydrogénation catalytique du nitrosocyanacétamide en aminocyanacétamide est du platine dans une quantité de 2% à 10% par rapport au carbone.

13. Procédé selon la revendication 1, 8, 9, 10 ou 11, **caractérisé en ce que** la quantité de catalyseur utilisée pour la réaction d'hydrogénation catalytique du nitrosocyanacétamide en aminocyanacétamide est de 1% à 30% par rapport au nitrosocyanacétamide, de préférence de 2% à 10% par rapport au nitrosocyanacétamide.

14. Procédé selon la revendication 1, 8, 9, 10, 11, 12 ou 13, **caractérisé en ce que** du nitrosocyanacétamide est transformé en aminocyanacétamide par une réaction d'hydrogénation catalytique avec de l'hydrogène à une pression de 1 bar à 100 bars, de préférence de 1 bar à 10 bars et à une température entre 0°C et 80°C, de préférence entre 20°C et 50°C.
